# FASCICULE DE BREVET EUROPEEN

(11) **EP 0 654 283 B1**
(45) Date de publication et mention de la délivrance du brevet: **14.04.1999**
(21) Numéro de dépôt: 94500190.7
(22) Date de dépôt: 22.11.1994
(51) Int. Cl.: A61M 25/10

(54) **Cathéter intra-aortique pour perfusion et conservation rénale**
Intraaortaler Katheter zur Perfusion und Konservation der Niere
Intra-aortic catheter for renal perfusion and conservation

(30) Priorité: 22.11.1993 ES 9302434
(43) Date de publication de la demande: 24.05.1995
(73) Titulaire: Anaya Fernandez de Lomana, Eugenio Fernando, Madrid (ES)
(72) Inventeur: Anaya Fernandez de Lomana, Eugenio Fernando, Madrid (ES)
(74) Mandataire: Urizar Anasagasti, Jesus Maria

(56) Documents cités:
- EP-A- 0 364 799
- US-A- 4 943 277
- US-A- 5 108 364

## Description

On décrit pour la première fois un dispositif qui comprend un cathéter à double ballon, à implantation intra-aortique au niveau de l'hile rénal, dont la finalité est de diagnostiquer, prévenir et traiter l'insuffisance rénale aiguë secondaire à hipoperfusion et, en cas de mort par arrêt du coeur, pour pouvoir conserver sur place les deux reins pour leur utilisation ultérieure comme greffes rénales.

L'objectif de l'implantation de ce cathéter est triple:
a) enregistrer instantanément et continuellement des variations hémodynamiques et des données biochimiques au niveau de l'hile rénal; b) maintenir une pression de perfusion rénale optimale (supérieure ou égale à 100 mmHg) tant qu'il y a une activité cardiaque et administrer directement au niveau rénal une substance quelconque afin de prévenir une insuffisance rénale aiguë chez le patient; et c) conserver sur place les deux reins pour leur utilisation postérieure comme greffes rénales, en cas de mort par arrêt du coeur.

Le cathéter à ballon pour contre-pulsation aortique est une technique thérapeutique habituelle dans l'assistance circulatoire mécanique du choc cardiogénique afin de maintenir la fonction ventriculaire gauche. Dans cette technique on introduit le cathéter par l'artère fémorale jusqu'à l'aorte thoracique descendante. En utilisant un électrocardiogramme (ECG) pour la synchronisation, le ballon de 30 à 40 cm se gonfle durant la diastole et se dégonfle immédiatement avant l'éjection ventriculaire gauche. La raison de la contre-pulsation est d'augmenter le flux sanguin coronaire en augmentant la pression de perfusion diastolique (gonflage du ballon) et de diminuer les demandes d'oxygène par le myocarde et d'améliorer la dépense cardiaque, en réduisant la post-charge (dégonflage du ballon). Les effets hémodynamiques de la contre-pulsation consistent à: augmenter la pression diastolique, avec élévation du flux coronaire; augmenter la dépense cardiaque (10-20%), et diminuer la pression de remplissage diastolique ventriculaire gauche. Les contre-indications sont la dissection ou anévrismes de l'aorte thoracique ou abdominale.

Un autre des cathéters à ballon d'implantation intra-aortique visait l'obtention de reins pour greffes chez des cadavres par arrêt du coeur. Le premier à avoir eu cette idée fut Wilson Se en 1968, cependant, ce cathéter n'avait qu'un seul ballon et lors de la perfusion sur place, le liquide de perfusion partait aux extrémités. Le cathéter à double ballon fut introduit pour la première fois par Banowski Lh. et ses collaborateurs, mais l'application de cette technique en clinique fut réalisée par García-Rinaldi R et ses collaborateurs en 1975, lequel communiqua dix cas de greffes rénales fonctionnant, en provenance de donneurs cadavres par arrêt du coeur.

Dans les pays où la mort cérébrale est admise (le coeur battant encore), les organes pour les greffes sont obtenus à partir de ce type de cadavres, et c'est pourquoi l'utilisation de ces cathéters s'est peu répandue; mais elle est toutefois largement acceptée dans les cas où la mort cérébrale n'est pas admise, comme c'est le cas de certains pays européens et du Japon.

C'est sans doute le travail paru récemment chez Clin. Transplantation (1993) par Itsuo Yokoyama et collaborateurs de l'Université de Nagoya (Japon), qui présente la plus grande expérience parue jusqu'à maintenant en utilisant le double ballon pour l'obtention de reins pour greffes. Il s'agit de 119 donneurs par arrêt du coeur où la survivance pour le récepteur est de 95,0% et de 93,0% pour respectivement un et cinq ans, et la survivance de la greffe est de 85,0% et de 72,7% également pour un et cinq ans. Tous ces résultats sont superposables aux meilleurs résultats obtenus de donneurs cadavres à coeur battant. Dans tous les cas, l'implantation du cathéter se fait par dissection de l'artère fémorale, aprés l'arrêt cardiaque. Une fois introduit le cathéter dans l'aorte, sa mise en place se fait selon la technique de "pull-back", c'est à dire, en gonflant les ballons et par retrait, en tirant du cathéter vers le bas, le ballon inférieur s'emboîtant au niveau de la bifurcation aorto-iliaque. Le temps moyen à partir du moment de l'arrêt cardiaque jusqu'à ce que commence la perfusion rénale (temps d'ischémie chaude) était de 12 minutes.

Le cathéter que l'on décrit ici est un cathéter intra-aortique à deux ballons, d'implantation par voie percutanée à travers l'artère fémorale, dans l'aorte abdominale, au niveau de la région rénale, à fonctionnement synchronique ou indépendant entre les deux ballons, dont la finalité est de maintenir la pression de perfusion rénale adéquate afin d'éviter une défaillance rénale aiguëe chez les patients à faible dépense cardiaque par choc cardiogénique; et également, d'enregistrer les variations analytiques dans d'autres cas d'hypofusion rénale. C'est sans doute un avantage clinique remarquable, mais ce type de cathéter en offre également d'autres très importants, à savoir:
- il permet l'enregistrement instantané et continu de la température, la pression artérielle et le flux sanguin au niveau parahilaire rénal, ce qui peut apporter de très nombreuses observations sur la physiologie rénale concernant les variations de la circulation rénale.
- la prise directe de sang au niveau des artères rénales peut donner lieu à l'étude de différentes substances qui influent sur la circulation rénale en situation de choc (rénine, vasopressine, prostaglandines, etc ...; voie efférente).
- le contact direct avec la circulation artérielle rénale peut servir également pour administrer une substance quelconque pharmacologique, vaso-active ou de toute autre catégorie qui change la circulation intrarénale et puisse mettre au clair des inconnues qui sont encore obscures dans de telles circonstances (voie afférente).
- avec le contrôle et le maintien de la pression de perfusion rénale s'ouvre une espérance de rétablissement chez les patients ayant eu un choc cardiogénique, dont la mortalité est trés haute.
- et, ceci est encore plus important, grâce à ce cathéter, on peut obtenir une nouvelle source de donneurs rénaux.

Bien que cette finalité pourrait être étendue à la conservation d'autres organes, tels que le foie ou le pancréas, de telles applications ne font pas partie de l'invention telle que revendiquée, et l'invention s'adresse à la conservation de reins pour leur greffe postérieure, chez des patients à insuffisance rénale terminale. Bien que la conservation sur place de ce type de donneurs a déjà été démontrée, cependant ce nouveau cathéter apporte les avantages suivants:
1. implantation de celui-ci par voie percutanée lorsque le coeur bat et n'est pas arrêté;
2. monitoring de la circulation artérielle pré-rénale au niveau aortique (hémodynamique, biochimique et autres);
3. contrôle pharmacologique rénal pour prévenir une défaillance rénale aiguëe durant la période de choc;
4. le temps d'ischémie chaude (temps à partir du moment où le coeur s'arrête jusqu'au moment où la perfusion rénale commence avec le liquide de circulation) est pratiquement zéro; ceci est très important car c'est l'un des principaux facteurs qui influent sur la fonctionnalité immédiate ou non de la greffe rénale;
5. avoir la possibilité de pouvoir apporter directement au rein toute substance vaso-active ou immunosuppresseur avec le liquide de perfusion (voie afférente).

A part ces avantages de conservation, le principal apport de ce cathéter est celui de pouvoir être une nouvelle source de reins pour la greffe. Pour se faire une idée de l'énorme portée que cela peut avoir, nous devons savoir qu'il y a actuellement en Espagne environ 14.500 patients à insuffisance rénale chronique soumis à un programme de dialyse à un coût approximatif de quelques 60.000 millions de pesetas/an. Sur ce total, 6.000 patients se trouvent sur la liste d'attente active, alors qu'on ne greffe que quelques 1.300/an, les autres greffes ne pouvant se faire par manque de donneurs (bien que l'Espagne soit un pays ayant l'un des plus grand nombre de greffes par million d'habitants/an de l'Europe).

Les avantages que ce genre de source peut apporter à certains pays tel que le Japon où il existe actuellement plus de 100.000 patients en dialyse sont insoupçonnables, car il n'y a pratiquement pas de greffe du fait que la mort cérébrale du donneur n'est pas admise.

Or, il ne faut pas oublier non plus que l'utilisation de ce cathéter peut apporter une autre série d'avantages économiques très importantes, si l'on tient compte des aspects suivants:
- Le nombre de patients ayant subi un choc par hypoperfusion susceptibles de bénéficier de ce cathéter, représente environ plus de 10-15% de tous ceux qui entrent dans un Service d'Urgence Coronaire ou de Surveillance d'urgence. Etant donné les caractéristiques cliniques de ceux-ci, leurs soins sanitaires sont très onéreux et le taux de mortalité est très élevé. L'utilisation du cathéter intra-aortique rénal peut diminuer considérablement ces très grands frais, car il permet d'écourter le temps moyen de séjour, le rétablissement étant plus rapide, et d'éviter le traitement par hémodialyse ou hémofiltration en prévenant l'insuffisance rénale aiguë.
- C'est une nouvelle source de donneurs pour la greffe rénale, à savoir les cadavres par arrêt du coeur, et évite l'épineux problème moral-éducatif-légal de la mort cérébrale qui rend si difficile actuellement le don d'organes; économiquement, c'est aussi un pas de géant; en effet chaque donneur représente une économie pour la société de plus de 7 millions de Pesetas, car un patient en dialyse représente des frais d'environ 3,5 millions/an. Ce simple fait justifierait déjà que tout hôpital recevant des patients d'urgence et comptant des services de réanimation de médecine intensive ou coronaire dispose du cathéter en question.

On comprendra mieux le fonctionnement du cathéter de la présente invention à l'aide de la description suivante, qui est réalisée sur la base des dessins représentés sur les plans annexes, sur lesquels:
La figure 1 montre une vue schématique du cathéter introduit dans l'aorte, situé en position opérationnelle et prêt à fournir toutes sortes d'informations hémodynamiques et biochimiques par voie efférente.

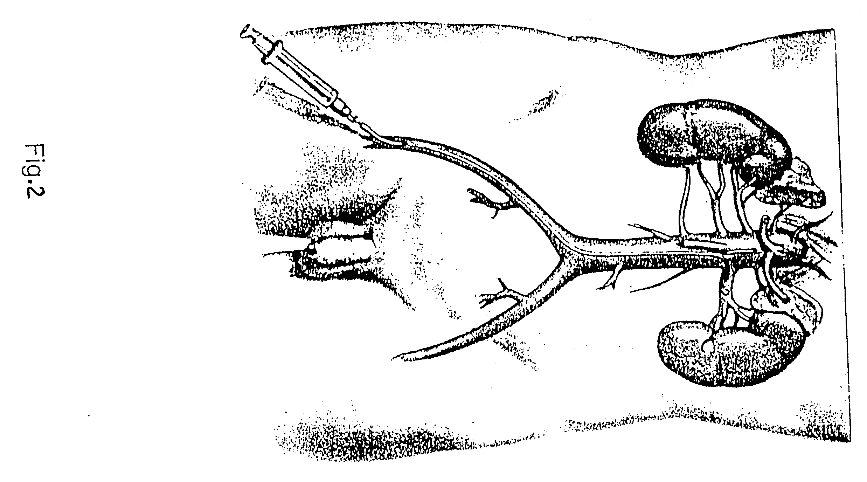

La figure 2 montre chez un patient la place et la façon de poser le cathéter décrit ici.
Les figures 3 et 4 représentent respectivement l'évolution que subit le cathéter lorsque la pression artérielle diminue dans la zone et lorsque le coeur s'est définitivement arrêté de manière irréversible et qu'il s'agit d'isoler et de conserver les reins du donneur.
Les figures 5, 6 et 7 montrent en section diamétrale trois types différents de cathéters, la figure 7 représentant un cathéter réalisé selon la présente invention.

Le cathéter de la présente invention est un tube de silicone ou de tout autre matériau utilisé habituellement pour les cathéters interartériels d'environ 25-50 cm de longueur et de 4 à 16 French de diamètre. Ce cathéter a trois ou quatre voies selon la technique utilisée pour son implantation, deux ballons qui se gonflent en obturant l'artère à ces points; et une région perméable intermédiaire.

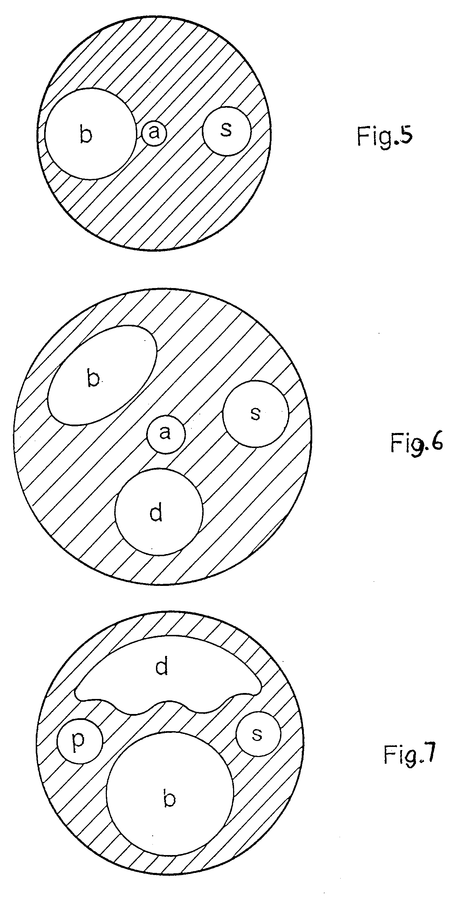

Sur une première réalisation, montrée sur la figure 5, le cathéter en question dispose des voies suivantes:
- voie (a); à travers cette voie on introduit le guide de fil métallique ou "poil" selon la technique percutanée de Seldinger. On peut se passer de cette technique si l'implantation se fait à travers un dilatateur-cliquet adapté au cathéter.
- voie (b); elle communique avec la zone perméable parahilaire-renale (voie efférente et afférente).
- voie des capteurs (s); communique également avec la zone hilaire rénale; sa finalité est la détection de la température et de la pression sanguine.

Cette première réalisation est employée dans le cas des patients où l'on souhaite le monitoring de toutes les constantes vitales au niveau rénal et, en fonction de cette information, pouvoir envisager d'éventuelles mesures thérapeutiques.

La réalisation montrée sur la figure 6 inclue, outre les voies citées ci-dessus, une quatrième voie (d) qui communique avec un ballon distal (D), qui sera placé sous les artères rénales.

Enfin, dans l'exemple de réalisation montré sur la figure 7, on a remplacé la voie pour l'introduction d'un guide métallique pour l'introduction d'un cathéter (a), par une autre voie (p) qui communique avec un ballon proximal (P), qui se situe au-dessus des artères rénales et qui a pour but, en conjonction avec le ballon distal (D), d'isoler les reins en cas d'arrêt cardiaque irréversible.

Evidemment, d'autres réalisations sont encore possibles. Une alternative est un cathéter à cinq voies, comprenant outre les voies ci-dessus, la voie pour l'introduction du guide métallique d'implantation. Une autre alternative comprendra la voie de capteurs à travers la voie (b) qui communique avec la zone perméable (B).

Comme nous l'avons indiqué, dans cette dernière réalisation il y a également deux ballons.
- Un ballon proximal (P) . Il est situé à l'extrémité proximale du cathéter, communique directement avec la voie (p) et sera implanté juste au-dessus des artères rénales. Son diamètre est tel que, lorsqu'il est gonflé, il obstrue totalement la circulation aortique.
- Un ballon distal (D) . Il est situé dans la partie distale ou caudale du cathéter. Il communique directement avec la voie (d) et doit se trouver sous les artères rénales. Il a une double mission: a) en cas de faible dépense cardiaque, il se gonflera et se dégonflera soit de manière isolée, soit par un mécanisme de contre-pulsation, dans le but de maintenir une pression de perfusion rénale adéquate, et b) en cas d'arrêt cardiaque irréversible il se gonflera synchroniquement avec le ballon proximal pour obstruer totalement la circulation aortique infra-rénale.

La région perméable (B) constitue une partie du cathéter située entre les deux ballons, juste dans la région parahilaire rénale, perméable à travers de nombreux orifices et en communication directe avec la voie (b) et a pour mission de contrôler instantanément les variations hémodynamiques et biochimiques (voie efférente) à ce niveau et, d'autre part, de pouvoir administrer un médicament quelconque directement dans la circulation rénale (voie afférente).

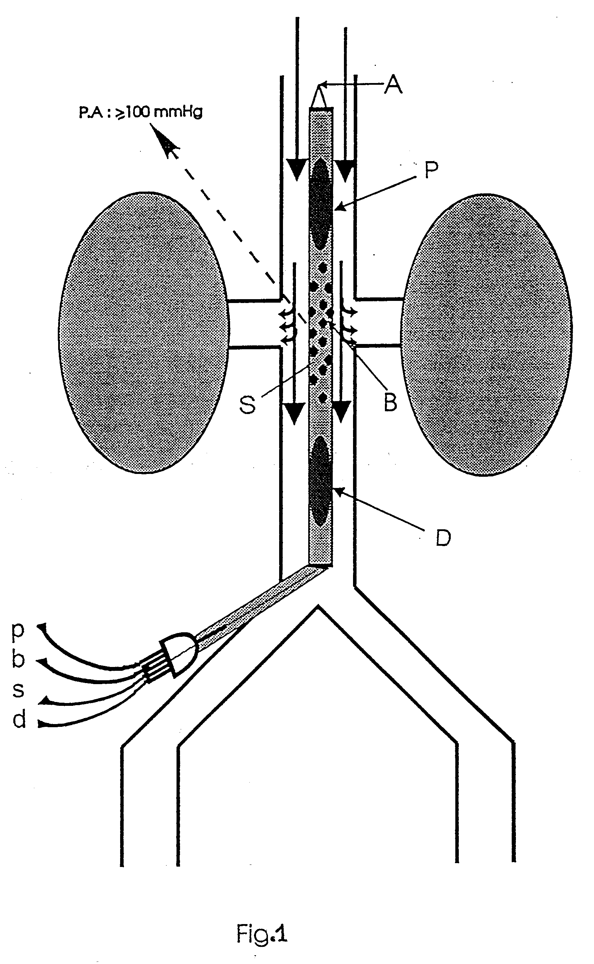

L'implantation du cathéter peut se faire selon la technique Selinger, caractérisant l'artère fémorale. On peut introduire le cathéter soit à travers le guide métallique, par la voie (a) tel que représenté sur les figures 5 et 6, soit directement au moyen d'un dilatateur-cliquet de diamètre adaptable au cathéter, comme dans le cas de la figure 7. Une fois introduit, sa mise en place devra être telle que le ballon proximal (P) et le ballon distal (D) se trouvent respectivement au-dessus et au-dessous des artères rénales. Pour sa vérification, il faudra utiliser un contrôle radiologique de contraste ou un autre moyen pour vérifier que la circulation rénale, y compris les artères anomales polaires, reste toujours entre les deux ballons (Fig. 1).

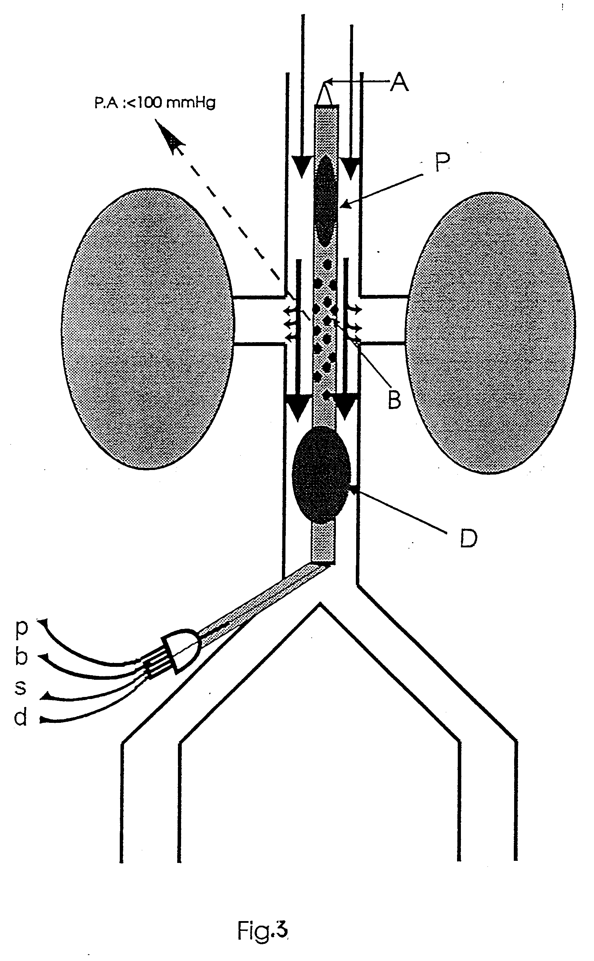

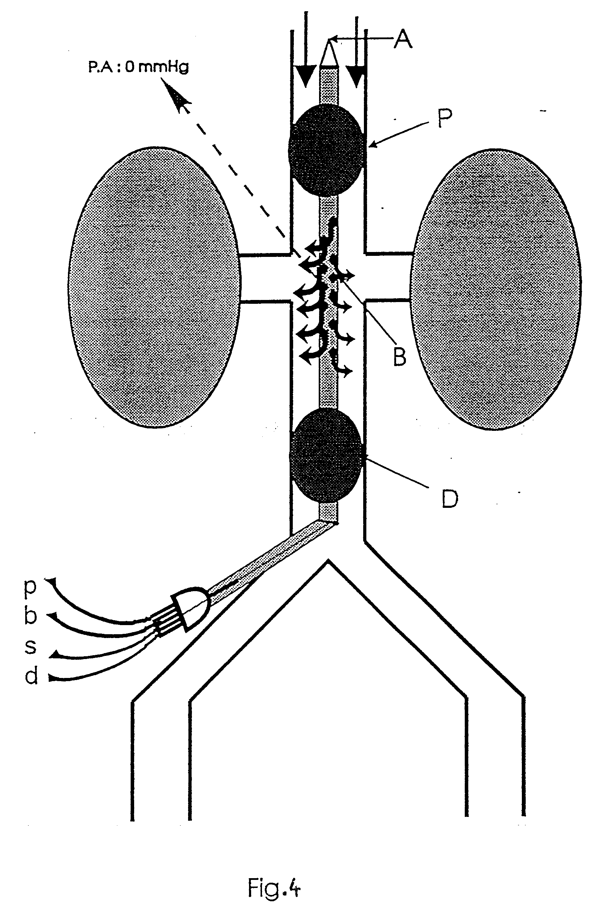

Les différentes terminaisons du cathéter sont connectées à un moniteur qui contrôlera et réalisera les différentes missions pour lesquelles le cathéter a été conçu. Ce moniteur comprendra les éléments suivants:
1.- Electrocardiographe. Cet appareil peut être incorporé au moniteur ou disposera d'une connexion à un électrocardiographe extérieur pour le monitoring permanent du patient.
2.- Groupe hémodynamique et biochimique. L'une des dérivations de la terminaison (s) présente une série de capteurs, avec une sortie dans la partie perméable parafilaire rénale, qui, connectés à des transducteurs électroniques adéquats fournissent la température et la pression artérielle pour obtenir un monitoring instantané et continu de la pression artérielle au niveau de la région parafilaire rénale.
   A travers la voie (b) on extrait une petite quantité de sang pour pouvoir déterminer, à travers un analyseur, instantanément et continuellement les analytiques suivantes:
   - la gazométrie (pH, pCO2, pO2, saturation 02, excès de bases et bicarbonate, etc);
   - une série de paramètres hématologiques: hématocrite (Hto), hémoglobine (Hb), leucocyte, plaquettes;
   - paramètres biochimiques: glucose, créatinine en plasma (Crp), urée; ions (Na, K, Mg, Ca ...);
   - de manière non instantanée, d'autres substance telles que les enzymes (GOT, GPT, CPK, MB), molécules vaso-actives ou niveaux pharmacologiques.
3. Groupe de régulation de gonflement des ballons. Les deux terminaisons (p) et (d) qui correspondent aux ballons proximal (P) et distal (D) respectivement, se connecteront indistinctement à un groupe de pompage qui règle le gonflement des différents ballons. Il y a deux circonstances différentes pour que le fonctionnement de ces ballons s'active, à savoir: en cas de choc cardiogénique à faible dépense cardiaque et en cas d'arrêt cardiaque irréversible.
   a.- En choc cardiogénique, avec une pression artérielle moyenne au niveau parafilaire rénal (PAMR) inférieure environ à 80 mmHg enregistrée par la zone perméable du cathéter, le ballon distal commence à se gonfler afin de maintenir une PAMR entre 100-130 mmHg (Fig. 3). Le mécanisme de gonflement de ce ballon peut se réaliser soit de manière régulière et proportionnelle à la PAMR, soit à travers un mécanisme de contre-pulsation synchronisé avec le ECG, en se gonflant durant la diastole et en se dégonflant immédiatement avant l'éjection ventriculaire gauche. Durant cette situation clinique, le ballon proximal demeure totalement inactif.
   b.- En cas d'arrêt cardiaque: Le groupe de gonflement s'active dans la situation suivante: ECG plat ou fibrillation ventriculaire et PAMH de zéro. Avant de s'activer, et durant 5 secondes, une alarme sonore-visuelle fonctionnera. Le mécanisme d'activation sera différent s'il existe ou non des manoeuvres de rappel (massage cardiaque). Dans le premier cas, seul le ballon distal se gonflera proportionnellement à la PAMH avec le massage. Si le patient sort de cette situation et le coeur récupère son activité, le mécanisme de ce ballon continuera de fonctionner comme s'il n'y avait pas eu d'arrêt cardiaque; ayant fourni durant cet arrêt une large information analytique de toutes les variables analytiques et de gazométrie, ce qui aidera au futur rétablissement du patient. Dans le cas où l'arrêt cardiaque est irréversible, le mécanisme d'action est semblable à la phase d'arrêt cardiaque sans rappel à la vie. Automatiquement et par sécurité également, il disposera d'un dispositif manuel avant que les deux ballons supérieur et inférieur se gonflent pour obstruer totalement la circulation aortique au-dessus et au-dessous de la circulation rénale, le groupe de pompe de perfusion commençant à fonctionner synchroniquement (Fig. 4).
4. Groupe de pompage pour administration de tout type de médicaments ou de sérums, tant que le patient est en vie, ou dans le cas d'arrêt cardiaque irréversible, il servira de perfusion pour la conservation des deux reins. La partie perméable parafilaire du cathéter est connectée directement à une pompe de perfusion qui à une double mission:
   a.- tant qu'il n'y a pas de risque imminent de mort, cette voie est idéale pour administrer des substances vasoactives, des médicaments ou des immunodépresseurs sélectifs;
   b.- lorsque survient la mort du patient, il transfuse du liquide de perfusion rénale à quelques O°C, pour conserver les deux reins, à une température d'environ 4°C. Pour obtenir une perfusion à O°C, ce liquide, qui sera conservé préalablement en réfrigérateur à cette température, passera par un système en forme de serpentin submergé dans de la glace ou à travers une chambre à neige carbonique; en option, il y a un thermomètre à aiguille à usage percutané et pour enregistrement intra-rénal de la température, celui-ci peut être incorporé au moniteur ou être indépendant.

   En cas d'augmentation de la pression de perfusion en raison du volume de liquide fourni, on pourra par l'un des cathéters veineux centraux (jugulaire, sous-clavier ou fémoral) effectuer une exsanguination proportionnelle à la pression de perfusion rénale en synchronisant la pompe de perfusion à une autre d'exsanguination qui extrait un débit de sang égal à celui du liquide qui est transfusé. Cette pompe d'exsanguination doit porter un détecteur d'hémoglobine pour que, lorsque le liquide exsanguiné manque de cette substance, le circuit se ferme avec du liquide de perfusion rénale exclusivement. Le volume de liquide à transfuser pour la bonne conservation des reins du point de vue biologique est de 2-3 litres. Le reste du volume que l'on transfuse ne sert qu'à maintenir la température rénale à 40°C, ce qui est en rapport avec le temps d'attente pour l'extraction et le milieu environnant.
   Une fois terminée l'extraction rénale, le cathéter sera retiré, et, après une stérilisation adéquate, il pourra être réutilisé.
5.- De plus, cet équipement dispose d'un enregistrement informatique où sont enregistrées toutes les variables hémodynamiques, hématologiques et biochimiques existant à chaque instant; ainsi, l'équipement constitue une "boîte noire", qui conserve toutes les circonstances qui se seraient produites chez le patient pendant que le cathéter était dans son organisme et connecté à l'équipement. Cette fonction aura une importante maximale pour connaître et analyser divers phénomènes qui se produisent durant toute opération ou dans la mort; il pourra également apporter des données de valeur juridique pour connaître et évaluer le travail de l'équipe médicale qui a traité le patient.

## Revendications

1. Dispositif pour perfusion et conservation rénale, comprenant un cathéter intra-aortique, formé d'un tube qui comprend une série de voies finissant dans différents points tout au long de celui-ci, caractérisé en ce que le cathéter comprend les voies suivantes: une première voie (b) qui communique avec une région perméable (B), laquelle région possède de nombreux orifices et occupe une zone intermédiaire du cathéter et qui, une fois le cathéter implanté, reste située juste dans la région parafilaire rénale; une seconde voie de capteurs (s) qui finit également dans la région parafilaire rénale; une voie complémentaire qui finit dans le bout du cathéter et qui permet d'introduire à travers elle un guide métallique pour son placement selon la technique percutanée; une troisième voie dite distale (d), en considérant le terme distal par rapport au sens de la circulation sanguine dans l'aorte, qui communique avec un ballon (D) situé dans la partie distale ou caudale du cathéter et capable d'obstruer la circulation dans l'aorte en se gonflant; et une quatrième voie dite proximale (p) qui communique directement avec un ballon proximal (P) situé dans le bout du cathéter et qui s'implante au dessus des artères rénales et a un diamètre tel que, en se gonflant, il obstrue aussi complètement la circulation aortique; et en ce que le dispositif comprend un équipement de monitoring auquel sont connectées les terminaisons disponibles ou voies, ledit équipement de monitoring comprenant : 1) un électrocardiographe incorporé au moniteur propre de l'équipement ou indépendant, 2) un groupe hémodynamique et biochimique, connecté à une série de capteurs (S) qui déterminent la pression artérielle et la température; ainsi que des moyens pour extraire à travers la première voie (b), qui communique avec la région perméable, un petit échantillon de sang avec lequel on détermine instantanément la gazométrie, une série de paramètres hématologiques et biochimiques, et avec un décalage, d'autres déterminations biochimiques et pharmacologiques d'intérêt, 3) un groupe connecté aux voies (p,d) proximale et distale, qui règle et effectue le gonflement des ballons, en produisant premièrement le gonflement du ballon distal lorsqu'il détecte une baisse de la pression parafilaire rénale ou un arrêt cardiaque réversible, tandis que le gonflement du ballon proximal se produit lorsqu'il se produit un arrêt cardiaque irréversible; 4) un groupe de pompage de perfusion, qui est connecté à la terminaison qui termine dans la zone perméable, qui permet de transfuser du liquide directement aux reins, lorsque le patient est vivant et qui sert, une fois qu'il y a un arrêt cardiaque irréversible et que les deux ballons se sont gonflés, à transfuser du liquide de conservation dans les deux reins et qui comprend un système d'exsanguination synchronisé avec cette pompe de perfusion; et 5) des moyens d'enregistrement sur un support informatique, de toutes les variations du monitoring au niveau de l'hile rénal.

## Claims

1. A device for renal perfusion and conservation comprising an intra-aortic catheter formed by a tube comprising a series of vias terminating in different points along the former, characterised in that the catheter comprises the following paths: a first path (b) communicating with a permeable region (B); said region has numerous holes and occupies an intermediate zone of the catheter and which, once the catheter is implanted, remains located in the renal para-filiform region; a second path for a sensing device(s), likewise terminating in the para-filiform region; a complementary path terminating in the end of the catheter and which permits the introduction of a metallic guide through it to fit it following the percutaneous method; a third path called distal (d), considering the term distal in relation to the direction of blood flow in the aorta, which communicates with the balloon (D) located in the distal or caudal part of the catheter and capable of obstructing the flow in the aorta by inflating; and a fourth path called proximal (p), communicating directly with a proximal balloon (P) located at the end of the catheter and which is implanted above the renal arteries and at a diameter such that on inflating, it also completely obstructs the aortic flow; and in that the device comprises a monitoring equipment to which the available terminations or paths are connected, said monitoring equipment comprises: (1) an electrocardiograph incorporated to the equipment or independent monitor itself, (2) an haemo-dynamic and biochemical group, connected to a series of sensing device(s) which determine the blood pressure and temperature as well as the means to extract through the first path (b), communicating with the permeable region, a small blood sample, with which the gasometry, a series of haematological and biochemical parameters, is determined, and with a difference compared with other biochemical and pharmacological determinations of interest, (3) a group connected to the proximal and distal paths (p,d), which regulate and inflate the balloons, producing the inflating of the distal balloon when it detects a renal parafiliform pressure reduction or a reversible cardiac standstill, whilst the inflating of the proximal balloon is produced when an irreversible cardiac standstill occurs; 4) a group of perfusion pumps connected to the termination finishing in the permeable zone, permitting the transfusion of liquid directly to the kidneys when the patient is still living and which serves, once there is an irreversible cardiac standstill and the two balloons are inflated, for the transfusion of the conservation liquid in the two kidneys and which comprises an exsanguination system synchronised with this perfusion pump and (5) recording means on a computerised support of all the monitoring variations at the renal hilium level.

## Patentansprüche

1. Vorrichtung zur Perfusion und Konservierung der Niere, wobei diese einen intraaortalen Katheter umfasst, der aus einer Röhre besteht, die eine Reihe von Bahnen umfasst, die auf verschiedenen Punkten auf der Länge der genannten Röhre enden, dadurch gekennzeichnet, dass der Katheter folgende Bahnen umfasst: eine erste Bahn (b), die Verbindung zu einer durchlässigen Region (B) hat, wobei die genannte Region zahlreiche Öffnungen aufweist und einen mittleren Bereich des Katheters einnimmt und nach dem Einsetzen des Katheters in der parafiliformen Nierengegend zu liegen kommt; eine zweite Bahn mit Fühlern (s), die ebenfalls in der parafiliformen Nierengegend enden; eine ergänzende Bahn, die am Ende des Katheters endet und die es ermöglicht durch sie eine metallische Führung einzuführen, um diese nach der perkutanen Technik zu plazieren; eine dritte Bahn (d), die distal genannt wird, wobei der Begriff distal in Bezug auf die Strömungsrichtung in der Aorta gemeint ist, und die mit einem Ballon (D) verbunden ist, der in dem distalen oder kaudalen Teil des Katheters liegt und der durch Aufblasen die Durchströmung der Aorta blockieren kann; und eine vierte Bahn (p), die als proximal bezeichnet wird und die direkt mit einem proximalen Ballon (P) in Verbindung steht, der am Ende des Katheters liegt, und der über den Nierenarterien eingesetzt wird und einen Durchmesser aufweist, der nach dem Aufblasen ebenfalls komplett die Durchströmung der Aorta blockiert; und dadurch, dass die Vorrichtung ein Monitoringgerät aufweist, an das die verfügbaren Enden oder Bahnen angeschlossen werden, wobei das genannte Monitoringgerät folgendes umfasst: 1) einen Elektrokardiographen, der im Monitor des Geräts eingebaut oder unabhängig sein kann, 2) ein hämodynamisches und biochemisches Aggregat, das an eine Reihe von Fühlern (s) angeschlossen ist, die den Blutdruck und die Temperatur messen, sowie Mittel, um über die erste Bahn (b), die mit der durchlässigen Region verbunden ist, eine kleine Blutprobe zu entnehmen, mit der sofort die Gasanalyse festgestellt werden kann, eine Reihe von hämatologischen und biochemischen Parametern, und mit einer Differenz zu anderen wichtigen biochemischen und pharmakologischen Messungen, 3) ein Aggregat, das an die proximale und die distale Bahn (p, d) angeschlossen ist, das das Aufblasen der Ballone regelt und durchführt, wobei zuerst der distale Ballon aufgeblasen wird, sobald eine Verringerung des parafiliformen Nierendrucks festgestellt wird oder ein reversibler Herzstillstand, wohingegen der proximale Ballon aufgeblasen wird, wenn es zu einem irreversiblen Herzstillstand kommt; 4) ein Perfusionspumpenaggregat, das an das Ende angeschlossen ist, welches in dem durchlässigen Bereich endet, das die Transfusion der Flüssigkeit direkt zu den Nieren ermöglicht, solange der Patient am Leben ist, und das dazu dient, nach einem irreversiblen Herzstillstand, wenn beide Ballone aufgeblasen sind, eine Transfusion der Konservierungsflüssigkeit zu den beiden Nieren durchzuführen, und das ein Ausblutungssystem umfasst, das mit dieser Perfusionspumpe synchronisiert ist; und 5) Mittel zur Aufzeichnung auf einem Datenträger, wobei alle Änderungen des Monitoring in Bezug auf den Nierenhilus festgehalten werden.
